(19) Europäisches Patentamt
European Patent Office
Office européen des brevets

(11) Veröffentlichungsnummer: **0 315 850 B1**

(12) **EUROPÄISCHE PATENTSCHRIFT**

(45) Veröffentlichungstag der Patentschrift: **15.07.92**

(51) Int. Cl.[5]: **A01N 43/653**, A01N 43/50, C07D 405/06

(21) Anmeldenummer: **88118076.4**

(22) Anmeldetag: **31.10.88**

# (54) Verfahren zur Beeinflussung des Pflanzenwachstums durch Azolylmethyloxirane.

(30) Priorität: **07.11.87 DE 3737888**

(43) Veröffentlichungstag der Anmeldung:
**17.05.89 Patentblatt 89/20**

(45) Bekanntmachung des Hinweises auf die Patenterteilung:
**15.07.92 Patentblatt 92/29**

(84) Benannte Vertragsstaaten:
**AT BE CH DE FR GB LI NL**

(56) Entgegenhaltungen:
**EP-A- 0 094 564**
**DE-A- 3 111 238**
**DE-A- 3 537 817**

(73) Patentinhaber: **BASF Aktiengesellschaft**
**Carl-Bosch-Strasse 38**
**W-6700 Ludwigshafen(DE)**

(72) Erfinder: **Karbach, Stefan, Dr.**
**Grundwiesenweg 44**
**W-6730 Neustadt(DE)**
Erfinder: **Janssen, Bernd, Dr.**
**Leuschnerstrasse 18 a**
**W-6700 Ludwigshafen(DE)**
Erfinder: **Recker, Hans-Gert, Dr.**
**3800 Parkview Lane Apt. 19c**
**Irvine, CA 92715(US)**
Erfinder: **Smuda, Hubert, Dr.**
**Rahmengasse 32**
**W-6900 Heidelberg(DE)**
Erfinder: **Meyer, Norbert, Dr.**
**Dossenheimer Weg 22**
**W-6802 Ladenburg(DE)**
Erfinder: **Jung, Johann, Prof. Dr.**
**Hardenburgstrasse 19**
**W-6703 Limburgerhof(DE)**
Erfinder: **Rademacher, Wilhelm, Dr.**
**Austrasse 1**
**W-6703 Limburgerhof(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein Verfahren zur Beeinflussung des Pflanzenwachstums, indem man den Boden, das Saatgut und/oder die Pflanzen mit einem Azolylmethyloxiran der allgemeinen Formel I

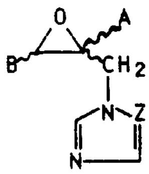

(I),

in der die Substituenten

A und B gleich oder verschieden sind und $C_1$-$C_4$-Alkyl, Phenyl, Naphthyl, Diphenyl oder durch ein bis drei gleiche oder verschiedene Reste aus der Gruppe Halogen, $C_1$-$C_4$-Alkyl, $C_1$-$C_4$-Alkoxy, $C_1$-$C_4$-Halogenalkyl, Nitro oder Phenoxy substituiertes Phenyl und

Z die CH-Gruppe oder Stickstoff bedeuten,

oder deren Metall- oder Säureadditionssalze, behandelt sowie Mittel zur Beeinflussung des Pflanzenwachstums, enthaltend bioregulatorisch wirksame Mengen eines Azolylmethyloxirans I.

Aus der EP-A-94 564 sind die Azolylmethyloxirane der allgemeinen Formel I bekannt; sie werden zur Verwendung als Arzneimittel, besonders als Antimykotika, und als Pflanzenschutzmittel, besonders als Fungizid, empfohlen. Andere Anwendungen dieser Verbindungen waren nicht bekannt.

Der Erfindung lag daher die Aufgabe zugrunde, die Azolylmethyloxirane für neue Anwendungsgebiete bereitzustellen.

Demgemäß wurde gefunden, daß die eingangs definierten Azolylmethyloxirane I sich zur Regulierung des Pflanzenwachstums eignen.

Im einzelnen haben die Substituenten in Formel I folgende Bedeutungen:

A und B unabhängig voneinander

- unverzweigtes oder verzweigtes $C_1$-$C_4$-Alkyl, wie Methyl, Ethyl, n-Propyl, iso-Propyl, n-Butyl, iso-Butyl, sec.-Butyl und tert.-Butyl,
- Phenyl,
- Naphthyl, wie 1-Naphthyl und 2-Naphthyl, bevorzugt 2-Naphthyl,
- Diphenyl, wie o-Diphenyl, m-Diphenyl und p-Diphenyl,
- durch ein, zwei oder drei Halogenatome substituiertes Phenyl, wie 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2,3-Difluorphenyl, 2,4-Difluorphenyl, 2,5-Difluorphenyl, 2,6-Difluorphenyl, 3,4-Difluorphenyl, 3,5-Difluorphenyl, 2,3-Difluorphenyl, 2,4-Dichlorphenyl, 2,5-Dichlorphenyl, 2,6-Dichlorphenyl, 3,4-Dichlorphenyl, 3,5-Dichlorphenyl, 2,3-Dibromphenyl, 2,4-Dibromphenyl, 2,5-Dibromphenyl, 2,6-Dibromphenyl, 3,4-Dibromphenyl, 3,5-Dibromphenyl, 2-Chlor-3-fluorphenyl, 3-Chlor-2-fluorphenyl, 2-Chlor-4-fluorphenyl, 4-Chlor-2-fluorphenyl, 2-Chlor-5-fluorphenyl, 5-Chlor-2-fluorphenyl, 2-Chlor-6-fluorphenyl, 3-Chlor-4-fluorphenyl, 4-Chlor-3-fluorphenyl, 3-Chlor-5-fluorphenyl, 2-Brom-3-fluorphenyl, 3-Brom-2-fluorphenyl, 2-Brom-4-fluorphenyl, 4-Brom-2-fluorphenyl, 2-Brom-5-fluorphenyl, 5-Brom-2-fluorphenyl, 2-Brom-6-fluorphenyl, 3-Brom-4-fluorphenyl, 4-Brom-3-fluorphenyl, 3-Brom-5-fluorphenyl, 2-Brom-3-chlorphenyl, 3-Brom-2-chlorphenyl, 2-Brom-4-chlorphenyl, 4-Brom-2-chlorphenyl, 2-Brom-5-fluorphenyl, 5-Brom-2-chlorphenyl, 4-Brom-6-chlorphenyl, 3-Brom-4-chlorphenyl, 4-Brom-3-chlorphenyl, 3-Brom-5-chlorphenyl,
- durch ein, zwei oder drei $C_1$-$C_4$-Alkylgruppen substituiertes Phenyl,
- durch ein, zwei oder drei $C_1$-$C_4$-Alkoxygruppen substituiertes Phenyl,
- durch ein, zwei oder drei $C_1$-$C_4$-Halogenalkylgruppen substituiertes Phenyl,
- durch ein oder zwei Nitrogruppen substituiertes Phenyl,
- durch ein, zwei oder drei Phenoxygruppen substituiertes Phenyl,
- durch Halogen und $C_1$-$C_4$-Alkyl zwei- oder dreifach substituiertes Phenyl,
- durch Halogen und $C_1$-$C_4$-Alkoxy zwei- oder dreifach substituiertes Phenyl,
- durch Halogen und $C_1$-$C_4$-Halogenalkyl zwei- oder dreifach substituiertes Phenyl,
- durch Halogen und Nitro zwei- oder dreifach substituiertes Phenyl,
- durch Halogen und Phenoxy zwei- oder dreifach substituiertes Phenyl,

- durch $C_1$-$C_4$-Alkyl und $C_1$-$C_4$-Alkoxy zwei- oder dreifach substituiertes Phenyl,
- durch $C_1$-$C_4$-Alkyl und $C_1$-$C_4$-Halogenalkyl zwei- oder dreifach substituiertes Phenyl,
- durch $C_1$-$C_4$-Alkyl und Nitro zwei- oder dreifach substituiertes Phenyl,
- durch $C_1$-$C_4$-Alkyl und Phenoxy zwei- oder dreifach substituiertes Phenyl,
- durch $C_1$-$C_4$-Alkoxy und $C_1$-$C_4$-Halogenalkyl zwei- oder dreifach substituiertes Phenyl,
- durch $C_1$-$C_4$-Alkoxy und Nitro zwei- oder dreifach substituiertes Phenyl,
- durch $C_1$-$C_4$-Alkoxy und Phenoxy zwei- oder dreifach substituiertes Phenyl,
- durch $C_1$-$C_4$-Halogenalkyl und Nitro zwei- oder dreifach substituiertes Phenyl,
- durch $C_1$-$C_4$-Halogenalkyl und Phenoxy zwei- oder dreifach substituiertes Phenyl,
- durch Nitro und Phenoxy zwei- oder dreifach substituiertes Phenyl,
- durch Halogen, $C_1$-$C_4$-Alkyl und $C_1$-$C_4$-Alkoxy dreifach substituiertes Phenyl,
- durch Halogen, $C_1$-$C_4$-Alkyl und $C_1$-$C_4$-Halogenalkyl dreifach substituiertes Phenyl
- durch Halogen, $C_1$-$C_4$-Alkyl und Nitro dreifach substituiertes Phenyl
- durch Halogen, $C_1$-$C_4$-Alkyl und Phenoxy dreifach substituiertes Phenyl
- durch Halogen, $C_1$-$C_4$-Alkoxy und $C_1$-$C_4$-Halogenalkyl dreifach substituiertes Phenyl
- durch Halogen, $C_1$-$C_4$-Alkoxy und Nitro dreifach substituiertes Phenyl
- durch Halogen, $C_1$-$C_4$-Alkoxy und Phenoxy dreifach substituiertes Phenyl
- durch Halogen, $C_1$-$C_4$-Halogenalkyl und Nitro dreifach substituiertes Phenyl
- durch Halogen, $C_1$-$C_4$-Halogenalkyl und Phenoxy dreifach substituiertes Phenyl
- durch Halogen, Nitro und Phenoxy dreifach substituiertes Phenyl
- durch $C_1$-$C_4$-Alkyl, $C_1$-$C_4$-Alkoxy und $C_1$-$C_4$-Halogenalkyl dreifach substituiertes Phenyl
- durch $C_1$-$C_4$-Alkyl, $C_1$-$C_4$-Alkoxy und Nitro dreifach substituiertes Phenyl
- durch $C_1$-$C_4$-Alkyl, $C_1$-$C_4$-Alkoxy und Phenoxy dreifach substituiertes Phenyl
- durch $C_1$-$C_4$-Alkyl, $C_1$-$C_4$-Halogenalkyl und Nitro dreifach substituiertes Phenyl
- durch $C_1$-$C_4$-Alkyl, $C_1$-$C_4$-Halogenalkyl und Phenoxy dreifach substituiertes Phenyl
- durch $C_1$-$C_4$-Alkyl, Nitro und Phenoxy dreifach substituiertes Phenyl
- durch $C_1$-$C_4$-Alkoxy, $C_1$-$C_4$-Halogenalkyl und Nitro dreifach substituiertes Phenyl
- durch $C_1$-$C_4$-Alkoxy, $C_1$-$C_4$-Halogenalkyl und Phenoxy dreifach substituiertes Phenyl
- durch $C_1$-$C_4$-Halogenalkyl, Nitro und Phenoxy dreifach substituiertes Phenyl.

Besonders bevorzugt sind die Azolylmethyloxirane I, in denen A für 4-Fluorphenyl oder 4-Chlorphenyl, B für Trifluormethylphenyl und Z für Stickstoff steht.

Geeignete Säuren zur Bildung von Salzen der Verbindungen I sind vorzugsweise Halogenwasserstoffsäuren, wie Chlorwasserstoffsäure und Bromwasserstoffsäure, insbesondere Chlorwasserstoffsäure, die mit den Verbindungen I besonders gut kristallisierende Salze bildet, ferner eignen sich Phosphorsäure, Salpetersäure, Schwefelsäure, mono- und bifunktionelle Carbonsäuren und Hydroxycarbonsäuren, wie Essigsäure, Oxalsäure, Maleinsäure, Bernsteinsäure, Fumarsäure, Weinsäure, Zitronensäure, Salicylsäure, Sorbinsäure, Milchsäure, sowie Sulfonsäuren, wie p-Toluolsulfonsäure und 1,5-Naphthalindisulfonsäure.

Säureadditionssalze sind beispielsweise die Chloride, Bromide, Sulfate, Nitrate, Phosphate, Oxalate oder Dodecyl-benzolsulfonate. Die Wirksamkeit der Salze geht auf das Kation zurück, so daß die Wahl des Anions beliebig ist. Metallkomplexe der Verbindungen I sind Verbindungen der allgemeinen Formel Ia

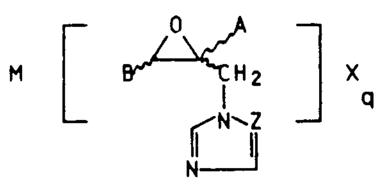
(Ia)

in der M ein Metall, z.B. Kupfer, Zink, Zinn, Mangan, Eisen, Kobalt oder Nickel, bevorzugt Kupfer bedeutet, W für das Anion einer anorganischen Säure steht, z.B. Chlorid, Sulfat, Phosphat oder Bromid, bevorzugt Chlorid und n und q 1, 2, 3 oder 4, bevorzugt 1 und 2, bedeuten.

Die Azolylmethyloxirane der allgemeinen Formel I lassen sich nach den Methoden, die in der EP-A-94 564 beschrieben sind, herstellen.

Die Verbindungen I enthalten 2 chirale Zentren und werden im allgemeinen in Form von Diastereomerengemischen erhalten. Diese lassen sich durch Diastereomerentrennung, aufgrund ihrer unterschiedlichen physikalischen Eigenschaften z.B. durch Umkristallisation oder Säulenchromatographie, auch HPLC, in diastereomerenreine Enantiomerenpaare trennen. Diese diastereomerenreinen Enantiomerenpaare können

durch bekannte Methoden der Racematspaltung, wie z.B. durch Umsetzung mit einer chiralen Hilfskomponente, Trennung der entstandenen Diastereomeren nach den oben genannten Methoden und anschließende Abspaltung der Hilfskomponente oder durch Säulenchromatographie, auch HPLC, an chiralen Säulen (Säulenfüllmaterial z.B. Kieselgel dotiert mit einem chiralen nicht herauswaschbaren festen Hilfsstoff), in enantiomerenreine Verbindungen I überführt werden.

Die Azolylmethyloxirane der allgemeinen Formel I können praktisch alle Entwicklungsstadien einer Pflanze verschiedenartig beeinflussen und werden deshalb als Wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist sich z.B. die Verringerung des Grasbewuchses an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung zur Hemmung des Längenwachstums und zur zeitlichen Veränderung des Reifeverlaufs bei Baumwolle. Damit wird ein vollständig mechanisiertes Beernten dieser wichtigen Kulturpflanzen ermöglicht.

Durch Anwendung der Verbindungen I kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit den Verbindungen I läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag, bezogen auf die Bodenfläche, erzielt werden kann.

B. Mit den Mitteln auf der Basis von Azolylmethyloxiranen I lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Die Azolylmethyloxirane der Formel I können Ertragssteigerungen durch Eingriffe in den pflanzlichen Stoffwechsel bzw. durch Förderung oder Hemmung des vegetativen und/oder des generativen Wachstums verursachen.

C. Mit den Azolylmethyloxiranen I lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Die Förderung der Ausbildung eines Trenngewebes zwischen der Blatt- und Sproßachse ist auch für ein gut kontrollierbares Entblättern von Nutzpflanzen z.B. Baumwolle wesentlich.

D. Mit den Azolylmethyloxiranen I kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.

- die Öffnungsweite der Stomata reduziert wird
- eine dickere Epidermis und Cuticula ausgebildet werden
- die Durchwurzelung des Bodens verbessert wird

das Mikroklima im Pflanzenbestand durch einen kompakteren Wuchs günstig beeinflußt wird.

Die erfindungsgemäß zu verwendenden Wirkstoffe können den Kulturpflanzen sowohl vom Samen her (als Saatgutbeizmittel) als auch über den Boden, d.h. durch die Wurzel sowie durch Spritzung über das Blatt zugeführt werden.

Infolge der hohen Verträglichkeit der Pflanzen für die Verbindungen I kann die Aufwandmenge stark variiert werden. Bei der Saatgutbehandlung werden im allgemeinen Wirkstoffmengen von 0,001 bis 50 g je Kilogramm Saatgut, vorzugsweise 0,01 bis 1 g, benötigt. Für die Blatt- und Bodenbehandlung sind im allgemeinen Gaben von 0,01 bis 10 kg/ha, bevorzugt 0,1 bis 5 kg/ha ausreichend.

Die Mittel auf der Basis von Azolylmethyloxiranen I können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylole, Toluol, Benzol), chlorierte Aromaten (z.B. Chlorbenzole), Paraffine (z.B. Erdölfraktionen), Alkohole (z.B. Methanol, Butanol), Amine (z.B. Ethanolamin), N,N-Dimethylformamid und Wasser; feste Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel oder sonstige oberflächenaktive Mittel, wie nichtionogene und anionische Emulgatoren (z.B. Polyoxyethylen-Fettalkohol-Ether, Alkylsulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, N,N-Dimethylformamid oder N-Methylpyrrolidon.

Die Formulierungen enthalten im allgemeinen zwischen 0,1 und 95 Gew.% Wirkstoff, vorzugsweise zwischen 0,5 und 90 Gew.%.

Die Formulierungen bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind:

I. 20 Gewichtsteile der Verbindung Nr. 8 werden in 3 Gewichtsteilen des Natriumsalzes der Diisobutylanphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II. 3 Gewichtsteile der Verbindung Nr. 131 werden mit 97 Gewichtsteilen feinteiligem Kaolin innig vermischt. Man erhält auf diese Weise ein Stäubemittel, das 3 Gew.% des Wirkstoffs enthält.

III. 30 Gewichtsteile der Verbindung Nr. 38 werden in einer Mischung aus 92 Gewichtsteilen pulverförmigem Kieselsäuregel und 8 Gewichtsteilen Paraffinöl, das auf die Oberfläche dieses Kieselsäuregels

gesprüht wurde, innig vermischt. Man erhält auf diese Weise eine Aufbereitung des Wirkstoffs mit guter Haftfähigkeit.

IV. 40 Gewichtsteile der Verbindung Nr. 43 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V. 20 Teile der Verbindung Nr. 43 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI. Man vermischt 90 Gewichtsteile der Verbindung Nr. 49 mit 10 Gewichtsteilen N-Methyl-$\alpha$-pyrrolidon und erhält eine Lösung, die zur Anwendung in Form kleinster Tropfen geeignet ist.

VII. 20 Gewichtsteile der Verbindung Nr. 19 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII. 20 Gewichtsteile der Verbindung Nr. 8 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX. 20 Gewichtsteile der Verbindung Nr. 8 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Die Mittel auf der Basis von Azolylmethyloxiranen I können in diesen Anwendungsformen auch zusammen mit anderen Wirkstoffen vorliegen, wie z.B. Herbiziden, Insektiziden, anderen Wachstumsregulatoren und Fungiziden, oder auch mit Düngemitteln vermischt und ausgebracht werden. Beim Vermischen mit Wachstumsregulatormischungen treten auch synergistische Effekte auf, d.h. die Wirksamkeit des Kombinationsproduktes ist größer als die addierten Wirksamkeiten der Einzelkomponenten.

Fungizide Wirkstoffe, die mit den Azolylmethyloxiranen I kombiniert werden können, sind beispielsweise:

Schwefel,
Dithiocarbamate und deren Derivate, wie
Ferridimethyldithiocarbamat,
Zinkdimethyldithiocarbamat,
Zinkethylenbisdithiocarbamat,
Manganethylenbisdithiocarbamat,
Mangan-Zink-ethylendiamin-bis-dithiocarbamat,
Tetramethylthiuramdisulfide,
Ammoniak-Komplex von Zink-(N,N-ethylen-bis-dithiocarbamat),
Ammoniak-Komplex von Zink-(N,N′-propylen-bis-dithiocarbamat),
Zink-(N,N′-propylen-bis-dithiocarbamat),
N,N′-Polypropylen-bis-(thiocarbamoyl)-disulfid;
Nitroderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat;
5-Nitro-isophthalsäure-di-isopropylester
heterocyclische Substanzen, wie
2-Heptadecyl-2-imidazolin-acetat,
2,4-Dichlor-6-(o-chloranilino)-s-triazin,
0,0-Diethyl-phthalimidophosphonothioat,
5-Amino-1-[bis-(dimethylamino)-phosphinyl]-3-phenyl-1,2,4-triazol,
2,3-Dicyano-1,4-dithioanthrachinon,

2-Thio-1,3-dithio-(4,5-b)-chinoxalin,
1-(Butylcarbamoyl)-2-benzimidazol-carbaminsäuremethylester,
2-Methoxycarbonylamino-benzimidazol,
2-(Furyl-(2))-benzimidazol,
2-(Thiazolyl-(4))-benzimidazol,
N-(1,1,2,2-Tetrachlorethylthio)-tetrahydrophthalimid,
N-Trichlormethylthio-tetrahydrophthalimid,
N-Trichlormethylthio-phthalimid,
N-Dichlorfluormethylthio-N′,N′-dimethyl-N-phenyl-schwefelsäurediamid,
5-Ethoxy-3-trichlormethyl-1,2,3-thiadiazol,
2-Rhodanmethylthiobenzthiazol,
1,4-Dichlor-2,5-dimethoxybenzol,
4-(2-Chlorphenylhydroazano)-3-methyl-5-isoxazolon,
Pyridin-2-thio-1-oxid,
8-Hydroxychinolin bzw. dessen Kupfersalz,
2,3-Dihydro-5-carboxanilido-6-methyl-1,4-oxathiin,
2,3-Dihydro-5-carboxanilido-6-methyl-1,4-oxathiin-4,4-dioxid,
2-Methyl-5,6-dihydro-4H-pyran-3-carbonsäure-anilin,
2-Methyl-furan-3-carbonsäureanilid,
2,5-Dimethyl-furan-3-carbonsäureanilid,
2,4,5-Trimethyl-furan-3-carbonsäureanilid,
2,5-Dimethyl-furan-3-carbonsäurecyclohexylamid,
N-Cyclohexyl-N-methoxy-2,5-dimethyl-furan-3-carbonsäureamid,
2-Methyl-benzoesäure-anilid,
2-Iod-benzoesäure-anilid,
N-Formyl-N-morpholin-2,2,2-trichlorethylacetal,
Piperazin-1,4-diylbis-(1-(2,2,2-trichlor-ethyl)-formamid,
1-(3,4-Dichloranilino)-1-formylamino-2,2,2-trichlorethan ,
2,6-Dimethyl-N-tridecyl-morpholin bzw. dessen Salze,
2,6-Dimethyl-N-cyclodedecyl-morpholin bzw. dessen Salze,
N-[3-(p-tert.-Butylphenyl)-2-methylpropyl]-cis-2,6-dimethylmorpholin,
N-[3-(p-tert.-Butylphenyl)-2-methylpropyl]-piperidin,
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol,
N-(n-Propyl)-N-(2,4,6-trichlorphenoxyethyl)-N′-imidazol-yl-harnstoff,
1-(4-Chlorphenoxy)-3,3-dimethyl-1-(1H-1,2,4-triazol-1-yl)-2-butanon,
1-(4-Chlorphenoxy)-3,3-dimethyl-1-(1H-1,2,4-triazol-1-yl)-2-butanol,
α-(2-Chlorphenyl)-α-(4-chlorphenyl)-5-pyrimidin-methanol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol,
sowie verschiedene Fungizide, wie
Dodecylguanidinacetat,
3-[3-(3,5-Dimethyl-2-oxycyclohexyl)-2-hydroxyethyl]-glutarimid,
Hexachlorbenzol,
DL-Methyl-N-(2,6-dimethyl-phenyl)-N-furoyl(2)-alaninat,
DL-N-(2,6-Dimethyl-phenyl)-N-(2′-methoxyacetyl)-alanin-methylester,
N-(2,6-Dimethylphenyl)-N-chloracetyl-D,L-2-aminobutyrolacton,
DL-N-(2,6-Dimethylphenyl)-N-(phenylacetyl)-alaninmethylester,
5-Methyl-5-vinyl-3-(3,5-dichlorphenyl)-2,4-dioxo-1,3-oxazolidin,
3-[3,5-Dichlorphenyl(-5-methyl-5-methoxymethyl]-1,3-oxazolidin-2,4-dion,
3-(3,5-Dichlorphenyl)-1-isopropylcarbamoylhydantoin,
N-(3,5-Dichlorphenyl)-1,2-dimethylcyclopropan-1,2-dicarbonsäureimid,
2-Cyano-[N-(ethylaminocarbonyl)-2-methoximino]-acetamid,
1-[2-(2,4-Dichlorphenyl)-pentyl]-1H-1,2,4-triazol,
2,4-Difluor-α-(1H-1,2,4-triazolyl-1-methyl)-benzhydrylalkohol.

Herstellungsbeispiel

I. Herstellung der Vorprodukte

2-Brommethyl-2-phenyl-3-(2-chlorphenyl)-oxiran

30 g 1-(2-Chlorphenyl)-2-phenyl-3-brom-prop-1-en wurden mit 23 g 3-Chlorperoxybenzoesäure in 500 ml Chloroform unter Rückfluß gekocht. Nach beendeter Reaktion wurde die Chloroform-Phase mit wäßriger Natriumhydrogencarbonat-Lösung und Wasser säurefrei gewaschen, über Natriumsulfat getrocknet und i.Vak. eingeengt. Aus dem Rückstand erhielt man 41,3 g (70,2 %) 2-Brommethyl-2-phenyl-3-(2-chlorphenyl)-oxiran, das anschließend weiterverarbeitet wurde.

II. Herstellung der Endprodukte

2-(1,2,4-Triazol-1-yl-methyl)-2-phenyl-3-(2-chlorphenyl)-oxiran

23 g 1,2,4-Triazol und 5 g Natriumhydrid (80 %ige Dispersion in Mineralöl) wurden in 150 ml N,N-Dimethylformamid suspendiert und bei Raumtemperatur mit einer Lösung aus 32 g 2-Brommethyl-2-phenyl-3-(2-chlorphenyl)-oxiran in 150 ml N,N-Dimethylformamid versetzt. Nach 8 h wurde die Reaktionslösung auf Wasser gegeben und mit Essigsäureethylester extrahiert. Die organische Phse wurde mit Wasser gewaschen, über Natriumsulfat getrocknet und das Lösungsmittel i.Vak. abgezogen. Aus dem entstandenen Diastereomerengemisch werden durch Ankristallisation aus Methyl-tert.-butylether 15 g cis-2-(1,2,4-Triazolyl-1-yl-methyl)-2-(phenyl)-3-(2-chlorphenyl)oxiran vom Schmelzpunkt 150 °C gewonnen. Aus den Mutterlaugen können 6,3 g trans-2-(1,2,4-Triazolyl-1-yl-methyl)-2-(phenyl)-3-(2-chlorphenyl)-oxiran vom Schmelzpunkt 117 °C isoliert werden.

Die in der nachstehenden Tabelle aufgeführten Verbindungen I können nach dem Herstellungsbeispiel aus entsprechenden Vorprodukten ohne weiteres erhalten werden und lassen eine gleichartige Wirkung erwarten. Die Nomenklatur cis- oder trans-Isomer bezieht sich auf die Stellung vom Azol zum Rest B bezüglich der C-C-Bindung des Oxiranrings.

Tabelle

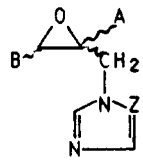

(I)

| Verbindung Nr. | A | B | Z | Isomer | Fp [°C] |
|---|---|---|---|---|---|
| 1 | Phenyl | 2-Chlorphenyl | N | cis | 159 |
| 2 | 4-Chlorphenyl | 2-Chlorphenyl | N | cis | 166 |
| 3 | 4-Diphenyl | 2-Chlorphenyl | N | cis | 191 |
| 4 | 2,4-Dichlorphenyl | 2-Chlorphenyl | N | cis | Harz |
| 5 | 2-Chlorphenyl | 2-Chlorphenyl | N | cis | |
| 6 | 2-Fluorphenyl | 2-Chlorphenyl | N | cis | 145-147 |
| 7 | 4-Tolyl | 2-Chlorphenyl | N | cis | 140 |
| 8 | 4-Fluorphenyl | 2-Chlorphenyl | N | cis | 136 |
| 9 | 3-Brom-4-fluorphenyl | 2-Chlorphenyl | N | cis | 129-130 |
| 10 | 4-Bromphenyl | 2-Chlorphenyl | N | cis | |
| 11 | 3,4-Dichlorphenyl | 2-Chlorphenyl | N | cis | |
| 12 | 4-tert.-Butylphenyl | 2-Chlorphenyl | N | cis | 122 |
| 13 | 3-Chlorphenyl | 2-Chlorphenyl | N | cis | |
| 14 | 3,5-Dichlorphenyl | 2-Chlorphenyl | N | cis | |
| 15 | 4-Phenoxyphenyl | 2-Chlorphenyl | N | cis | |
| 16 | 4-tert.-Butylphenyl | 2-Chlorphenyl | CH | cis | 123 |
| 17 | 4-Fluorphenyl | 2-Chlorphenyl | CH | cis | 119-122 |
| 18 | 4-Fluorphenyl | 2-Chlorphenyl | CH | trans | Harz |
| 19 | 4-Fluorphenyl | 2-Chlorphenyl | N | trans | Harz |
| 20 | 2-Fluorphenyl | 2-Chlorphenyl | CH | cis | 87 |
| 21 | 4-Chlorphenyl | 2-Chlorphenyl | CH | cis | 90- 92 |
| 22 | Phenyl | 2-Chlorphenyl | CH | cis | 85- 87 |
| 23 | 4-Chlorphenyl | 2-Fluorphenyl | N | cis | 140 |
| 24 | Phenyl | 2-Fluorphenyl | N | cis | 139 |
| 25 | 2-Fluorphenyl | 2-Fluorphenyl | N | cis | 128 |
| 26 | 2,4-Dichlorphenyl | 2-Fluorphenyl | N | cis | Harz |
| 27 | 4-Fluorphenyl | 2-Trifluormethylphenyl | N | cis | 110-112 |
| 28 | Phenyl | 4-Fluorphenyl | N | cis | 73 |
| 29 | 4-Fluorphenyl | 2-Trifluormethylphenyl | CH | cis | 133-135 |
| 30 | 4-Fluorphenyl | 2,4-Dichlorphenyl | N | cis | 127 |
| 31 | 4-Fluorphenyl | 3-Chlorphenyl | N | cis | Harz |
| 32 | 4-Fluorphenyl | 3-Chlorphenyl | CH | cis | Harz |

| Verbindung Nr. | A | B | Z | Isomer | Fp [°C] |
|---|---|---|---|---|---|
| 33 | 4-Chlorphenyl | 2-Chlor-4-fluorphenyl | CH | cis | 132 |
| 34 | 4-Chlorphenyl | 2-Chlor-4-fluorphenyl | N | cis | 143-145 |
| 35 | Phenyl | 2-Chlor-4-fluorphenyl | CH | cis | 117 |
| 36 | Phenyl | 2-Chlor-4-fluorphenyl | N | cis | 123 |
| 37 | 4-Fluorphenyl | 2-Chlor-4-fluorphenyl | CH | cis | 135 |
| 38 | 4-Fluorphenyl | 2-Chlor-4-fluorphenyl | N | cis | 139 |
| 39 | 4-Fluorphenyl | Phenyl | N | cis | 89- 92 |
| 40 | 2-Fluorphenyl | 2-Fluorphenyl | CH | cis | 79- 82 |
| 41 | Phenyl | 4-Trifluormethylphenyl | CH | cis | 106 |
| 42 | Phenyl | 4-Trifluormethylphenyl | N | cis | 132 |
| 43 | 4-Chlorphenyl | 4-Trifluormethylphenyl | N | cis | 135 |
| 44 | 4-Chlorphenyl | 4-Trifluormethylphenyl | CH | cis | 123 |
| 45 | 2,4-Dichlorphenyl | 3-Fluorphenyl | CH | cis | 125 |
| 46 | 4-Fluorphenyl | 4-Bromphenyl | N | cis | 119 |
| 47 | 4-Fluorphenyl | 3-Fluorphenyl | N | cis | 99 |
| 48 | 4-Fluorphenyl | 4-Fluorphenyl | N | cis | 69 |
| 49 | 4-Chlorphenyl | 2-Chlor-6-fluorphenyl | N | cis | 150 |
| 50 | Phenyl | 4-Bromphenyl | N | cis | 122 |
| 51 | Phenyl | 2-Chlor-6-fluorphenyl | N | cis | 158 |
| 52 | 4-Tolyl | 2-Fluorphenyl | N | cis | 127 |
| 53 | 4-Fluorphenyl | 2-Fluorphenyl | N | cis | 114 |
| 54 | 3-Brom-4-fluorphenyl | 2-Fluorphenyl | N | cis | 106 |
| 55 | 4-Diphenyl | 2-Fluorphenyl | N | cis | |
| 56 | 2-Chlorphenyl | 2-Fluorphenyl | N | cis | |
| 57 | 4-Bromphenyl | 2-Fluorphenyl | N | cis | |
| 58 | 3,4-Dichlorphenyl | 2-Fluorphenyl | N | cis | |
| 59 | 4-tert.-Butylphenyl | 2-Fluorphenyl | N | cis | |
| 60 | 3-Chlorphenyl | 2-Fluorphenyl | N | cis | |
| 61 | 3,5-Dichlorphenyl | 2-Fluorphenyl | N | cis | |
| 62 | 4-Phenoxyphenyl | 2-Fluorphenyl | N | cis | |
| 63 | Phenyl | 2-Bromphenyl | N | cis | 153 |
| 64 | 4-Bromphenyl | Phenyl | CH | cis | 152-153 |
| 65 | 4-Bromphenyl | 4-Chlorphenyl | CH | cis | 143-144 |
| 66 | Phenyl | 2,4-Dichlorphenyl | CH | cis | 103 |
| 67 | 4-Bromphenyl | 2,4-Dichlorphenyl | CH | cis | 107-108 |
| 68 | 2,4-Dichlorphenyl | 4-Chlorphenyl | CH | cis | 135 |

| Verbindung Nr. | A | B | Z | Salz | Isomer | Fp [°C] |
|---|---|---|---|---|---|---|
| 69 | 4-Chlorphenyl | Phenyl | CH | | cis | 138 |
| 70 | 4-Bromphenyl | 2,4-Dichlorphenyl | N | | cis | 133-134 |
| 71 | Methyl | 2,4-Dichlorphenyl | CH | | cis | 113-117,5 |
| 72 | Methyl | 2,4-Dichlorphenyl | CH | | trans | 98-104 |
| 73 | tert.-Butyl | 4-Chlorphenyl | N | | cis | 79- 86 |
| 74 | tert.-Butyl | 4-Chlorphenyl | CH | HCl | cis | 214-216 |
| 75 | tert.-Butyl | Phenyl | N | HCl | cis | 148 |
| 76 | tert.-Butyl | Phenyl | CH | | cis | 75 |
| 77 | tert.-Butyl | 2,4-Dichlorphenyl | N | | cis | 124 |
| 78 | tert.-Butyl | 2,4-Dichlorphenyl | CH | | cis | 95 |
| 79 | 4-Chlorphenyl | 4-tert.-Butylphenyl | CH | | trans | 160-162 |
| 80 | 4-Chlorphenyl | 4-tert.-Butylphenyl | N | | cis | 176-177 |
| 81 | 2,4-Dichlorphenyl | 4-tert.-Butylphenyl | CH | | cis | 132-134 |
| 82 | Methyl | 2,4-Dichlorphenyl | N | | cis | 105-108 |
| 83 | Methyl | 2,4-Dichlorphenyl | N | | trans | 80- 85 |
| 84 | Methyl | 2,4-Dichlorphenyl | N | | cis/trans =1:1 | 70- 81 |
| 85 | 4-tert.-Butylphenyl | 4-Chlorphenyl | CH | | cis | 100-102 |
| 86 | 4-tert.-Butylphenyl | 4-Chlorphenyl | N | | cis | 105-107 |
| 87 | 4-tert.-Butylphenyl | 2,4-Dichlorphenyl | CH | | cis | 101-113 |
| 88 | 4-tert.-Butylphenyl | 2,4-Dichlorphenyl | N | | cis | 108-111 |
| 89 | 4-Chlorphenyl | 3-Methoxyphenyl | CH | HCl | cis | 173 |
| 90 | 4-Chlorphenyl | 3-Methoxyphenyl | N | | cis | 77 |
| 91 | Phenyl | 2,4-Dichlorphenyl | N | | cis | 159-161 |
| 92 | 4-Chlorphenyl | 3-Trifluormethylphenyl | CH | | cis | 101-104 |
| 93 | 4-Chlorphenyl | 3-Trifluormethylphenyl | N | | cis | 107-109 |
| 94 | Phenyl | 3-Trifluormethylphenyl | CH | | cis | 77- 78,5 |
| 95 | Phenyl | 3-Trifluormethylphenyl | N | HCl | cis | 131-133 |
| 96 | Phenyl | Phenyl | CH | | cis | 108-110 |
| 97 | 4-Chlorphenyl | 4-Fluorphenyl | CH | | cis | 130-132 |
| 98 | Phenyl | 4-Chlorphenyl | CH | | cis | 105-106 |
| 99 | Phenyl | Phenyl | N | | cis | 116-118 |
| 100 | Phenyl | 4-Chlorphenyl | N | | cis | 114-115 |
| 101 | 4-Chlorphenyl | Phenyl | N | | cis | 106-110 |
| 102 | 4-Diphenyl | 4-Chlorphenyl | N | | cis | 163-165 |
| 103 | 4-Bromphenyl | Phenyl | N | | cis | 115-120 |
| 104 | 4-Bromphenyl | 4-Chlorphenyl | N | | cis | 115-120 |
| 105 | 4-Chlorphenyl | 4-Fluorphenyl | N | | cis | 112-117 |
| 106 | 4-Chlorphenyl | 4-Bromphenyl | N | | cis | 115-119 |
| 107 | 4-Chlorphenyl | 4-Bromphenyl | CH | | cis | 114-116 |

| Verbindung Nr. | A | B | Z | Salz | Isomer | Fp [°C] |
|---|---|---|---|---|---|---|
| 108 | 4-Chlorphenyl | 4-Bromphenyl | CH | | trans | 179-181 |
| 109 | 2,4-Dichlorphenyl | 4-Bromphenyl | CH | | cis | 135-139 |
| 110 | 4-Chlorphenyl | 4-Fluorphenyl | N | | trans | 219-223 |
| 111 | 4-Chlorphenyl | 4-Bromphenyl | N | | trans | 210-213 |
| 112 | 2,4-Dichlorphenyl | 4-Bromphenyl | N | | cis | 108-110 |
| 113 | 2,4-Dichlorphenyl | Phenyl | CH | | cis | Harz |
| 114 | 2,4-Dichlorphenyl | Phenyl | CH | | trans | 118-121 |
| 115 | 2,4-Dichlorphenyl | Phenyl | N | | cis | Harz |
| 116 | 4-Benzolsulfonyl-phenyl | 4-Chlorphenyl | CH | | cis | 193-195 |
| 117 | 4-Benzolsulfonyl-phenyl | 4-Chlorphenyl | CH | | trans | 204-205 |

| Verbindung Nr. | A | B | Z | Metall-komplex | Isomer | Fp [°C] |
|---|---|---|---|---|---|---|
| 118 | 4-Benzolsulfonyl-phenyl | 4-Chlorphenyl | N | | cis | 132-135 |
| 119 | 4-Benzolsulfonyl-phenyl | 4-Chlorphenyl | N | | trans | 175-177 |
| 120 | 2-Naphthyl | 2,4-Dichlorphenyl | CH | | cis | 135 |
| 121 | 2-Naphthyl | 2,4-Dichlorphenyl | N | | cis | 151 |
| 122 | 4-Chlorphenyl | 4-Chlorphenyl | CH | | cis | 115-120 |
| 123 | 4-Chlorphenyl | 4-Chlorphenyl | N | | cis | 105-108 |
| 124 | 2-Naphthyl | 4-Chlorphenyl | CH | | cis | 140 |
| 125 | 2-Naphthyl | 4-Chlorphenyl | N | | cis | 107 |
| 126 | 2,4-Dichlorphenyl | 4-tert.-Butylphenyl | CH | | trans | 142-143 |
| 127 | 3,4-Dichlorphenyl | 4-Bromphenyl | N | | cis | 130-134 |
| 128 | 4-Bromphenyl | Phenyl | CH | 1/2 CuCl | cis | 198-200 |
| 129 | 4-Chlorphenyl | 2-Chlorphenyl | N | 1/2 $CuCl_2$ | cis | 155 |
| 130 | 4-Fluorphenyl | 2-Chlorphenyl | N | 1/2 $CuCl_2$ | cis | 175 |
| 131 | 4-Chlorphenyl | 2-Fluorphenyl | N | 1/2 $CuCl_2$ | cis | 145 |
| 132 | Phenyl | 2-Chlorphenyl | N | 1/2 $CuCl_2$ | cis | 210 |

Anwendungsbeispiele

1. Verkürzung der Wuchshöhe

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser und einem Volumen von 500 ml angezogen.

Im Vorauflaufverfahren wurden die Testsubstanzen in wäßriger Aufbereitung am Tage der Einsaat auf das Saatbett gegossen. Im Nachauflaufverfahren wurden die zu prüfenden Substanzen in wäßriger Aufbereitung auf die Pflanzen gesprüht.

Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt.

Gleichlaufend zur Reduzierung des Längenwachstums steigt die Farbintensität der Blätter an. Der erhöhte Chlorophyllgehalt läßt eine ebenfalls erhöhte Photosynthese und damit eine erhöhte Ertragsbildung erwarten.

Als Vergleichssubstanz diente Chlormequatchlorid (CCC, 2-Chlorethyltrimethylammoniumchlorid).

Tabelle 1

| Sommergerste, Sorte "Aramir"; Vorauflauf-Bodenbehandlung | | |
|---|---|---|
| Verbindung Nr. | Konzentration in mg Wirkstoff/Gefäß | relative Wuchshöhen |
| unbehandelt | - | 100 |
| CCC | 6 | 86,1 |
| 27 | 6 | 25,7 |
| 43 | 6 | 43,1 |

Tabelle 2

| Rasen "Tiergarten Mischung"; Nachauflauf-Blattbehandlung | | |
|---|---|---|
| Verbindung Nr. | Konzentration in mg Wirkstoff/Gefäß | relative Wuchshöhen |
| unbehandelt | - | 100 |
| CCC | 1,5 | 104,1 |
| | 6 | 104,1 |
| 43 | 1,5 | 65,8 |
| | 6 | 38,4 |

Tabelle 3

| Sonnenblume "Sorex"; Nachauflauf-Blattbehandlung | | |
|---|---|---|
| Verbindung Nr. | Konzentration in mg Wirkstoff/Gefäß | relative Wuchshöhen |
| unbehandelt | - | 100 |
| CCC | 6 | 84,7 |
| 8 | 6 | 63,5 |
| 19 | 6 | 77,6 |
| 43 | 6 | 64,0 |
| 131 | 6 | 71,8 |

Tabelle 4

| Sommerraps "Petranova"; Nachauflauf-Blattbehandlung | | |
|---|---|---|
| Verbindung Nr. | Konzentration in mg Wirkstoff/Gefäß | relative Wuchshöhen |
| unbehandelt | - | 100 |
| CCC | 6 | 89,3 |
| 4 | 6 | 69,1 |
| 7 | 6 | 76,0 |
| 8 | 6 | 70,6 |
| 27 | 6 | 69,1 |
| 30 | 6 | 81,7 |
| 34 | 6 | 72,2 |
| 43 | 6 | 80,2 |
| 49 | 6 | 72,2 |
| 131 | 6 | 71,9 |

2. Reiskeimlingstest

Junge Reiskeimlinge (Sorte Bahia) wurden in einer Nährlösung kultiviert, die die jeweiligen Wirkstoffe in unterschiedlichen Konzentrationen enthielt. Nach sechs Tagen Kultur bei 25 °C im Dauerlicht wurde die Wirkstoffkonzentration bestimmt, die das Längenwachstum der zweiten Blattscheide um 50 % vermindert (= $kI_{50}$).
(W. Rademacher und J. Jung, Berichte aus dem Fachgebiet Herbologie, Heft 24, pp 127-134, Universität Hohenheim, 1983).

| Verbindung Nr. | $KI_{50}$ (molar) |
|---|---|
| CCC | $1,5 \times 10^{-2}$ |
| 7 | $1,8 \times 10^{-5}$ |
| 8 | $1,3 \times 10^{-6}$ |
| 17 | $6,5 \times 10^{-6}$ |
| 19 | $6,0 \times 10^{-5}$ |
| 30 | $6,4 \times 10^{-6}$ |
| 33 | $8,9 \times 10^{-6}$ |
| 34 | $2,8 \times 10^{-6}$ |
| 35 | $3,2 \times 10^{-5}$ |
| 36 | $7,3 \times 10^{-6}$ |
| 37 | $1,5 \times 10^{-5}$ |
| 38 | $1,2 \times 10^{-6}$ |
| 47 | $1,1 \times 10^{-6}$ |
| 48 | $8,1 \times 10^{-7}$ |
| 49 | $3,0 \times 10^{-6}$ |

3. Förderung des Kornertrags bei Gerste

Sommergerste der Sorte Aura wurde in 5 l-Mitscherlichgefäßen auf einem lehmigen Sandboden, dem ausreichend Nährstoffe zugegeben waren, angezogen. Die Bestandesdichte war 14 Pflanzen pro Gefäß. Alle zum Versuch gehörigen Pflanzen wurden mehrfach mit Fungiziden behandelt, um so einen Pilzbefall und damit eine Verfälschung der Ergebnisse weitgehend auszuschließen. Die Kultur der Pflanzen fand im Freien statt. Es erfolgte lediglich bisweilen ein Schutz gegen ungünstige Witterungsbedingungen.

Bei einer Wuchshöhe von ca. 38 cm wurden die Pflanzen mit einer wäßrigen Aufbereitung eines Azolylmethyloxirans I gleichmäßig besprüht (3,1 ml/Mitscherlichgefäß). Die Kornerträge sind in der folgenden Tabelle dargestellt und zeigen, daß zum Beispiel die Verbindung Nr. 8 zu einer deutlichen Ertragsverbesserung führt. Von der Versuchsdurchführung her kann ausgeschlossen werden, daß dieser Mehrertrag durch eine etwaige fungizide Wirkung der Testsubstanz oder durch die Vermeidung des Lagerns zustande gekommen sein kann.

| Verbindung Nr. | Aufwandmenge (mg Wirkstoff/Gefäß) | Ertrag [%] bez. auf 100 % der Kontrolle |
|---|---|---|
| 8 | 5 | 120 |
| 8 | 10 | 111 |
| 8 | 15 | 118 |

## Patentansprüche

1. Verfahren zur Beeinflussung des Pflanzenwachstums, dadurch gekennzeichnet, daß man den Boden, das Saatgut und/oder die Pflanzen mit einem Azolylmethyloxiran der allgemeinen Formel I

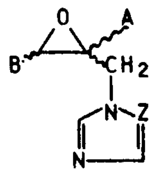

(I),

in der die Substituenten

A, B $C_1$-$C_4$-Alkyl, Phenyl, Naphthyl, Diphenyl oder durch ein bis drei Reste aus der Gruppe Halogen, $C_1$-$C_4$-Alkyl, $C_1$-$C_4$-Alkoxy, $C_1$-$C_4$-Halogenalkyl, Nitro oder Phenoxy substituiertes Phenyl und

Z die CH-Gruppe oder Stickstoff bedeuten

oder deren Metall- oder Säureadditionssalze, behandelt.

2. Verfahren zur Beeinflussung des Pflanzenwachstums nach Anspruch 1, dadurch gekennzeichnet, daß man den Boden, das Saatgut und/oder die Pflanzen mit 0,1 bis 95 Gew.% eines Azolylmethyloxirans der Formel I neben üblichen Trägerstoffen behandelt.

3. Verwendung der Azolylmethyloxirane der allgemeinen Formel I gemäß Anspruch 1 zur Beeinflussung des Pflanzenwachstums.

4. Verfahren zur Beeinflussung des Pflanzenwachstums, dadurch gekennzeichnet, daß man den Boden, das Saatgut und/oder die Pflanzen mit einem Azolylmethyloxiran der allgemeinen Formel I gemäß Anspruch 1 behandelt, in der A für 4-Fluorphenyl, B für 2-Trifluormethylphenyl und Z für Stickstoff steht.

5. Verfahren zur Beeinflussung des Pflanzenwachstums, dadurch gekennzeichnet, daß man den Boden, das Saatgut und/oder die Pflanzen mit einem Azolylmethyloxiran der allgemeinen Formel I gemäß Anspruch 1 behandelt, in der A für 4-Chlorphenyl, B für 4-Trifluormethylphenyl und Z für Stickstoff steht.

6. Mittel zur Beeinflussung des Pflanzenwachstums, gekennzeichnet durch einen wirksamen Gehalt an mindestens einem Azolylmethyloxiran der Formel I

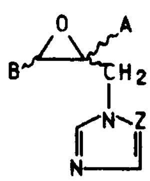

(I),

in der die Substituenten

A, B $C_1$-$C_4$-Alkyl, Phenyl, Naphthyl, Diphenyl oder durch ein bis drei Reste aus der Gruppe Halogen, $C_1$-$C_4$-Alkyl, $C_1$-$C_4$-Alkoxy, $C_1$-$C_4$-Halogenalkyl, Nitro oder Phenoxy substituiertes Phenyl und

Z die CH-Gruppe oder Stickstoff bedeuten

oder deren Metall- oder Säureadditionssalze.

7. Mittel nach Anspruch 6, gekennzeichnet durch einen wirksamen Gehalt an einem Azolylmethyloxiran der Formel I, worin A für 4-Fluorphenyl, B für 2-Trifluormethylphenyl und Z für Stickstoff steht.

8. Mittel nach Anspruch 6, gekennzeichnet durch einen wirksamen Gehalt an einem Azolylmethyloxiran der Formel I, worin A für 4-Chlorphenyl, B für 4-Trifluormethylphenyl und Z für Stickstoff steht.

9. Mittel zur Beeinflussung des Pflanzenwachstums gemäß den Ansprüchen 6 bis 8, enthaltend mindestens ein Azolylmethyloxiran der Formel I und einen flüssigen oder festen Trägerstoff sowie gegebenenfalls ein oder mehrere oberflächenaktive Mittel.

**Claims**

1. A method for influencing plant growth, which comprises treating the soil, the seed or the plants with an azolylmethyloxirane of the general formula I

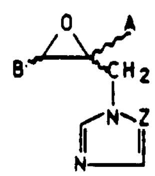

(I)

where A and B are each $C_1$-$C_4$-alkyl, phenyl, naphthyl or diphenyl or are each phenyl which is substituted by one to three radicals from the group consisting of halogen, $C_1$-$C_4$-alkyl, $C_1$-$C_4$-alkoxy, $C_1$-$C_4$-haloalkyl nitro and phenoxy, and Z is CH or nitrogen, or a metal salt or acid addition salt thereof.

2. A method for influencing plant growth as claimed in claim 1, wherein the soil, the seed or the plants are (is) treated with from 0.1 to 95% by weight of an azolylmethyloxirane of the formula I, together with conventional carriers.

3. Use of an azolylmethyloxirane of the general formula I as claimed in claim 1 for influencing plant growth.

4. A method for influencing plant growth, wherein the soil, the seed or the plants are(is) treated with an azolylmethyloxirane of the general formula I as claimed in claim 1, where A is 4-fluorophenyl, B is 2-trifluoromethylphenyl and Z is nitrogen.

5. A method for influencing plant growth, wherein the soil, the seed or the plants are(is) treated with an azolylmethyloxirane of the general formula I as claimed in claim 1, where A is 4-chlorophenyl, B is 4-trifluoromethylphenyl and Z is nitrogen.

6. An agent for influencing plant growth, containing an effective amount of one or more azolylmethyloxiranes of the formula I

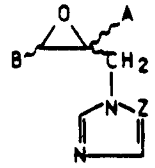

(I)

where A and B are each $C_1$-$C_4$-alkyl, phenyl, naphthyl or diphenyl or are each phenyl which is substituted by one to three radicals from the group consisting of halogen, $C_1$-$C_4$-alkyl, $C_1$-$C_4$-alkoxy, $C_1$-$C_4$-haloalkyl nitro and phenoxy, and Z is CH or nitrogen, or a metal salt or acid addition salt thereof.

**7.** An agent as claimed in claim 6, containing an effective amount of an azolylmethyloxirane of the formula I, where A is 4-fluorophenyl, B is 2-trifluoromethylphenyl and Z is nitrogen.

**8.** An agent as claimed in claim 6, containing an effective amount of an azolylmethyloxirane of the formula I, where A is 4-chlorophenyl, B is 4-trifluoromethylphenyl and Z is nitrogen.

**9.** An agent for influencing plant growth as claimed in any of claims 6 to 8, containing one or more azolylmethyloxiranes of the formula I, a solid or liquid carrier and, if desired, one or more surfactants.

**Revendications**

**1.** Procédé pour agir sur la croissance des végétaux, caractérisé en ce que l'on traite la terre, les semences et/ou végétaux par un azolylméthyloxiranne de formule générale I

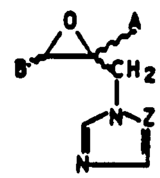

(I),

dans laquelle les symboles

A, B représentent des groupes alkyle en C1-C4, phényle, naphtyle, diphényle ou phényle portant un à trois substituants choisis parmi les halogènes, les groupes alkyle en C1-C4, alcoxy en C1-C4, halogénoalkyle en C1-C4, nitro ou phénoxy, et

Z représente le groupe CH ou l'azote,

ou par leurs sels métalliques ou leurs sels formés par addition avec des acides.

**2.** Procédé pour agir sur la croissance des végétaux selon la revendication 1, caractérisé en ce que l'on traite la terre, les semences et/ou les végétaux par 0,1 à 95 % en poids d'un azolylméthyloxiranne de formule I et des véhicules usuels.

**3.** Utilisation des azolylméthyloxirannes de formule générale I de la revendication 1, pour agir sur la croissance des végétaux.

**4.** Procédé pour agir sur la croissance des végétaux, caractérisé en ce que l'on traite la terre, les semences et/ou les végétaux par un azolylméthyloxiranne de formule généréle I de la revendication 1, dans laquelle A représente un groupe 4-fluorophényle, B un groupe 2-trifluorométhylphényle et Z l'azote.

**5.** Procédé pour agir sur la croissance des végétaux, caractérisé en ce que l'on traite la terre, les semences et/ou les végétaux par un azolylméthyloxiranne de formule générale I de la revendication 1, dans laquelle A représente un groupe 4-chlorophényle, B un groupe 4-trifluorométhylphényle et Z l'azote.

**6.** Produit pour agir sur la croissance des végétaux, caractérisé en ce qu'il contient une proportion efficace d'au moins un azolylméthyloxiranne de formule I

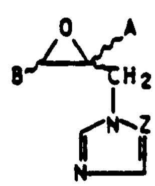

(I),

dans laquelle les symboles

- A, B représentent des groupes alkyle en C1-C4, phényle, naphtyle, diphényle ou phényle portant un à trois substituants choisis parmi les halogènes, les groupes alkyle en C1-C4, alcoxy en C1-C4, halogénoalkyle en C1-C4, nitro ou phénoxy, et
- Z représente le groupe CH ou l'azote,

ou de ses sels métalliques ou formés par addition avec des acides.

**7.** Produit selon la revendication 6, caractérisé en ce qu'il contient une proportion efficace d'un azolylméthyloxiranne de formule I dans laquelle A représente le groupe 4-fluorophényle, B le groupe 2-trifluorométhylphényle et Z l'azote.

**8.** Produit selon la revendication 6, caractérisé en ce qu'il contient une proportion efficace d'un azolylméthyloxiranne de formule I dans laquelle A représente un groupe 4-chlorophényle, B un groupe 4-trifluorométhylphényle et Z l'azote.

**9.** Produit pour agir sur la croissance des végétaux selon les revendications 6 à 8, contenant au moins un azolylméthyloxiranne de formule I et un véhicule liquide ou solide et le cas échéant un ou plusieurs agents tensioactifs.